(19) Europäisches Patentamt
European Patent Office
Office européen des brevets

(11) EP 3 137 530 B1

(12) # EUROPEAN PATENT SPECIFICATION

(45) Date of publication and mention of the grant of the patent:
**27.05.2020 Bulletin 2020/22**

(21) Application number: **15722107.8**

(22) Date of filing: **28.04.2015**

(51) Int Cl.:
***C08G 18/75*** *(2006.01)*
***C08G 18/34*** *(2006.01)*
***C08G 18/42*** *(2006.01)*
***C08G 18/08*** *(2006.01)*
***C08G 18/40*** *(2006.01)*
***C08J 5/18*** *(2006.01)*
***A61K 8/87*** *(2006.01)*
***C08J 7/18*** *(2006.01)*
***C08G 18/67*** *(2006.01)*
***C08G 18/66*** *(2006.01)*
***C08G 18/81*** *(2006.01)*
***C08G 18/48*** *(2006.01)*
***C08G 18/70*** *(2006.01)*
***A61Q 3/02*** *(2006.01)*
***C09D 175/16*** *(2006.01)*

(86) International application number:
**PCT/EP2015/059207**

(87) International publication number:
**WO 2015/165902 (05.11.2015 Gazette 2015/44)**

(54) **CURABLE AQUEOUS POLYURETHANE DISPERSIONS MADE FROM RENEWABLE RESOURCES.**

AUS ERNEUERBAREN RESSOURCEN HERGESTELLTE HÄRTBARE WÄSSRIGE POLYURETHANDISPERSIONEN

DISPERSIONS AQUEUSES DE POLYURÉTHANNE DURCISSABLE PRÉPARÉES À PARTIR DE RESSOURCES RENOUVELABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2014 US 201461986170 P**

(43) Date of publication of application:
**08.03.2017 Bulletin 2017/10**

(73) Proprietor: **Arkema France**
**92700 Colombes (FR)**

(72) Inventors:
- **KLANG, Jeffrey**
**West Chester,Pennsylvania 19382 (US)**
- **LU, Jin**
**West Chester, Pennsylvania 19382 (US)**
- **VAPPALA, Indu**
**Exton, Pennsylvania 19341 (US)**
- **HE, Yuhong**
**Honey Brook, Pennsylvania 19344 (US)**

(74) Representative: **Schaefer, Anne-Sophie et al**
**Arkema France**
**Département Propriété Industrielle**
**420, rue d'Estienne d'Orves**
**92705 Colombes Cedex (FR)**

(56) References cited:
**EP-A1- 2 644 634**
**WO-A1-2006/047434**
**US-A- 6 162 506**
**US-A1- 2008 112 909**
**US-A1- 2012 276 028**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 137 530 B1

## Description

The present invention relates to nail polish formulations based on curable polyurethane aqueous dispersions made from renewable resources. More specifically, the present invention relates to nail polish formulations based on curable polyurethane aqueous dispersions made from 1,4:3,6-dianhydrohexitol-based polyols.

Polyurethane dispersions find many uses in industry. For example, polyurethane dispersions may be used to coat wood, plastic, metal, glass, fibers, textiles, leather, stone, concrete and other substrates to provide protection against mechanical, chemical and/or environmental effects. Polyurethane dispersions may also be used for adhesives, sealants, inks and other applications, including cosmetic applications, such as nail polishes and the like.

Polyurethane dispersions are typically produced by first forming a polyurethane pre-polymer, which comprises terminal groups, such as isocyanate (NCO) groups, which undergo subsequent chain extension reactions. The polyurethane pre-polymer or simply pre-polymer is generally formed by reacting an excess of an isocyanate with a polyol to form the isocyanate-terminated pre-polymer.

The polyol typically provides flexibility and elasticity to the polyurethane. Attempts have been made to use renewable resources, such as, for example vegetable oils, but such attempts have resulted in soft polyurethanes that lack the mechanical hardness and chemical resistance desired in many applications.

Attempts have also been made to use polyurethane dispersions using isosorbide. Due to its structure, isosorbide leads to brittleness in polyurethanes.

Chang et al. ("Linseed-oil-based waterborne UV/air dual-cured wood coatings", Progress in Organic Coatings 76 (2013) 1024-1031) disclose an UV curable polyurethane dispersion made from linseed oil, a vegetable oil.

Xia et al. ("Soybean Oil-Isosorbide-Based Waterborne Polyurethane-Urea Dispersions", ChemSusChem 2011, 4, 386-391) disclose a conventional polyurethane dispersion made from a soybean oil derivative and isosorbide. The isosorbide is used as a monomeric chain extender and not as part of the polyol. The polyurethane dispersion disclosed by Xia et al. is not UV curable.

WO 2011/047369 discloses a polyurethane dispersion made using an epoxidized or partially epoxidized triglyceride, such as epoxidized soy oil.

U.S. Patent No. 8,106,148 discloses polyester polyols that incorporate isosorbide for use in powder coatings. The polyester polyols include non-renewable ingredients such as phthalates to achieve the desired properties. U.S. Patent No. 8,106,148 does not disclose UV curable compositions.

WO 2011/098272 discloses polyurethane dispersions based on renewable diisocyanates, including isosorbide-based diisocyanates. WO 2011/098272 does not disclose UV curable polyurethane dispersions.

U.S. Patent Application Publication No. 2005/0143549 discloses polyurethanes formed from polyester polyols. The polyester polymers are formed from a dimer fatty acid and/or a dimer fatty acid diol and may additionally be formed from a 1,4:3,6-dianhydrohexitol, such as isosorbide. The polyurethane is used as a hot melt adhesive.

U.S. Patent No. 6,372,201 discloses a nail varnish containing an aqueous dispersion of particles of acrylic polymer, a first organic solvent having a boiling point greater than or equal to 225°C and a second organic solvent having a boiling point ranging from 70°C to 180°C.

U.S. Patent No. 6,267,950 discloses a nail varnish comprising an aqueous dispersion of a film-forming polymer and an associative polyurethane. The associative polyurethane is a nonionic block copolymer comprising hydrophilic sequences and hydrophobic sequences. The film-forming polymer may be chosen from free radical polymers, polycondensates and polymers of natural origin.

U.S. Patent No. 5,716,603 discloses an aqueous acrylic resin crosslinked with difunctional acrylated urethane oligomers for use in nail polish compositions. The acrylic resin crosslinked with difunctional acrylated urethane oligomers is formed by the polymerization of : (1) a difunctional acrylated urethane oligomer; (2) an $\alpha,\beta$-ethylenically unsaturated carboxylic acid monomer containing 3 to 10 carbon atoms ; (3) an acrylate ester of a specified formula and (4) a methacrylate ester of a specified formula.

U.S. Patent Application No. 2007/0243149 discloses an aqueous binder system for nail varnishes based on nitrocellulose-containing polyurethane-polyurea dispersions.

Therefore, there is a need for a polyurethane dispersion based on renewable resources that provides a polyurethane having desirable mechanical strength and chemical resistance that is not soft or brittle and particularly suitable for nail polish formulations.

The present invention is as defined in the claims.

The present invention relates to nail polish formulations based on specific curable aqueous polyurethane dispersions.

One subject of the present invention is a nail polish formulation comprising :

- a curable aqueous polyurethane dispersion ;
- a photoinitiator ;

- optionally a leveling agent and
- optionally a thickener

wherein the formulation is free of non-reactive solvents and wherein said curable aqueous polyurethane dispersion is formed by the reaction of a) at least one polyol component comprising a1) a non-ionic polyol, a2) at least one polyol bearing at least one ionic or potentially ionic group comprising an acid group or salt thereof and a3) at least one ethylenically unsaturated monoalcohol or polyol, with b) at least one polyisocyanate, wherein the number of isocyanate (NCO) groups of the at least one polyisocyanate b) is in excess with respect to the number of hydroxyl (OH) groups of the at least one polyol component a),
with said at least one polyol component a1) comprising carbon atoms from renewable resources, said acid group of polyol a2) being at least partly neutralized and said reaction being followed in a second step by an extension reaction with an isocyanate-reactive chain extender c).

Said aqueous curable polyurethane dispersion is prepared by a method comprising :

i) reacting at least one polyol component a) comprising a1) a non-ionic polyol, a2) at least one polyol bearing at least one ionic or potentially ionic group comprising an acid group or salt thereof and a3) at least one ethylenically unsaturated monoalcohol or polyol with b) at least one polyisocyanate, wherein the number of isocyanate (NCO) groups of the at least one polyisocyanate b) is in excess with respect to the number of hydroxyl (OH) groups of said at least one polyol component a), wherein said at least one polyol component a) comprises carbon atoms from renewable resources,
ii) neutralizing at least partly the acid group of said polyol a2) with a neutralizing agent and agitating to obtain a polyurethane pre-polymer aqueous dispersion and
iii) chain extending said pre-polymer of step ii) by reacting with an isocyanate-reactive chain extender c).

So, said curable aqueous polyurethane dispersion is formed by the reaction of a) at least one polyol component comprising a1) a non-ionic polyol, a2) at least one polyol bearing at least one ionic or potentially ionic group comprising an acid group or salt thereof and a3) at least one ethylenically unsaturated monoalcohol or polyol, with b) at least one polyisocyanate, wherein the number of isocyanate (NCO) groups of the at least one polyisocyanate b) is in excess with respect to the number of hydroxyl (OH) groups of the at least one polyol component a), wherein said at least one polyol component a) comprises carbon atoms from renewable resources, wherein said acid group of polyol a2) being in at least partly neutralized form and said reaction being continued in a second step by an extension reaction with an isocyanate-reactive chain extender c).

Another aspect of the present disclosure relates to a nail polish formulation comprising :

- an aqueous polyurethane dispersion formed in a reaction comprising at least one polyol component comprising carbon atoms from renewable resource ;
- a photoinitiator ;
- optionally a leveling agent and
- optionally a thickener

wherein the formulation is free of non-reactive solvent.

Figure 1 is a schematic of a synthesis process for forming a UV-curable polyurethane dispersion using a bio-based polyol, dispersion suitable for said nail polish formulation as defined according to the present invention.

One aspect of the present disclosure relates to a nail polish formulation based on specific curable polyurethane aqueous dispersion formed by reacting at least one polyol component with at least one polyisocyanate, wherein the at least one polyol component comprises carbon atoms from renewable resources.

As used herein, the phrase "carbon atoms from renewable resources" refers to carbon atoms that are derived, sourced from or made from naturally renewable resources, such as, for example, bio-mass or plant-based sources.

The first subject of the present invention relates to a nail polish formulation comprising :

- a curable aqueous polyurethane dispersion ;
- a photoinitiator ;
- optionally a leveling agent and
- optionally a thickener

with said formulation being free of non-reactive solvents and said curable aqueous polyurethane dispersion being formed by the reaction of a) at least one polyol component comprising a1) a non-ionic polyol, a2) at least one polyol bearing at least one ionic or potentially ionic group comprising an acid group or salt thereof and a3) at least one ethylenically

unsaturated monoalcohol or polyol, with b) at least one polyisocyanate, wherein the number of isocyanate (NCO) groups of the at least one polyisocyanate b) is in excess with respect to the number of hydroxyl (OH) groups of the at least one polyol component a),
with said at least one polyol component a1) comprising carbon atoms from renewable resources, said acid group of polyol a2) being at least partly neutralized, and said reaction being followed in a second step by an extension reaction with an isocyanate-reactive chain extender c).

According to one embodiment, the at least one non-ionic polyol a1) comprises a polyol formed by reacting a 1,4:3,6-dianhydrohexitol in a polycondensation polymerization reaction with at least one diacid or higher functional carboxylic acid, by reacting a 1,4:3,6-dianhydrohexitol with a hydroxyl functional monocarboxylic acid or by reacting a 1,4:3,6-dianhydrohexitol with caprolactone. In particular, said 1,4:3,6-dianhydrohexitol is selected from the group consisting of isosorbide, isomannide and isoidide and preferably is isosorbide.

According to another option, the at least one non-ionic polyol a1) comprises a polyol formed by reacting a 1,4:3,6-dianhydrohexitol in a polycondensation polymerization reaction with at least one diol or polyol and at least one diacid or higher functional carboxylic acid, wherein the diol or polyol is selected from the group consisting of 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1 ,3-propanediol, diethylene glycol, triethylene glycol, dipropylene glycol, tripropylene glycol, 1,4- and 1,6-dimethylolcylcohexane, $C_{36}$-dimer diol, hydroxypivaloyl hydroxyl pivalate and ethoxylated and/or propoxylated derivatives thereof. The said at least one diacid or higher functional carboxylic acid may be selected from the group consisting of malonic acid, succinic acid, maleic acid, fumaric acid, itaconic acid, glutaric acid, citric acid, adipic acid, pimelic acid, sebacic acid, dodecanedioic acid, phthalic acid, isophthalic acid, terephthalic acid, naphthalene dicarboxylic acid, $C_{36}$-dimer fatty acids, $C_{54}$-trimer fatty acids (triacid) trimellitic acid, pyromellitic acid and anhydrides thereof.

According to another embodiment, the said non-ionic polyol a1) is formed using a 1,4:3,6-dianhydrohexitol as a hydroxyl functional initiator molecule in a ring opening polymerization of lactide.

More particularly, said monoalcohol or polyol a3) is an isocyanate-reactive ethylenically unsaturated monoalcohol or polyol selected from the group consisting of polyester (meth)acrylates, epoxy (meth)acrylates, polyether (meth)acrylates, polyurethane (meth)acrylates and hydroxyl group-containing (meth)acrylates.

In at least one embodiment, the polyol component a) comprises a1) a non-ionic polyol, a2) at least one polyol bearing at least one ionic group from acids and a3) at least one ethylenically unsaturated monoalcohol or polyol.

According to at least one embodiment, the non-ionic polyol a2) is formed by reacting a 1,4:3,6-dianhydrohexitol in a condensation polymerization reaction with other renewable diols or diacids or higher functional carboxylic acids or as an initiator for ring opening polymerization of lactide.

The 1,4:3,6- dianhydrohexitol may be selected from the group consisting of isosorbide, isomannide and isoidide, more preferably isosorbide.

In accordance with at least one embodiment, the non-ionic polyol is formed by reacting a 1,4:3,6-dianhydrohexitol with at least one diacid or higher functional carboxylic acid. As used herein, the phrase "higher functional carboxylic acid" refers to a carboxylic acid having an acid functionality of at least 3.

In at least one embodiment, the non-ionic polyol may be formed by a condensation polymerization reaction with a 1,4:3,6-dianhydrohexitol and at least one other diol or polyol and at least one diacid or higher functional carboxylic acid. Examples of other diols include, but are not limited to, 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, $C_{36}$-dimer diol, hydroxypivaloyl hydroxy pivalate and ethoxylated and/or propoxylated derivatives of the above. Suitable tri- and higher hydroxyl functional components include : glycerol, trimethylolpropane, trimethylolethane, pentaerythritol, di-glycerol, di-trimethyolpropane, di-pentaerytritol, sorbitol and ethoxylated and/or propoxylated derivatives of the above.

In at least one embodiment, the non-ionic polyol is formed by reacting a 1,4:3,6-dianhydrohexitol with at least one diacid or higher functional carboxylic acids. According to at least one embodiment, the diacid or higher functional carboxylic acid is selected from malonic acid, succinic acid, maleic acid, fumaric acid, itaconic acid, glutaric acid, adipic acid, pimelic acid, sebacic acid, dodecanedioic acid, phthalic acid, isophthalic acid, terephthalic acid, naphthalene dicarboxylic acid, $C_{36}$-dimer fatty acids, $C_{54}$-trimer fatty acids, trimellitic acid, pyromellitic acid and the anhydride derivatives of the above.

According to at least one embodiment, the non-ionic polyol a2) is formed using a 1,4:3,6-dianhydrohexitol as a hydroxyl functional initiator molecule in a ring opening polymerization of lactide (cyclic diester of lactic acid). Other monomers capable of undergoing ring opening polymerization, such as lactones, can also be used to make copolymer polyols. Suitable lactones include $\alpha,\alpha$-dimethyl-$\beta$-propiolactone, $\lambda$-butyrolactone and $\varepsilon$-caprolactone.

According to at least one embodiment, the amount of 1,4:3,6-dianhydrohexitol in the polyol component may be used to control the glass transition temperature (Tg) and hardness of the resulting polyurethane. For example, the

polyol may contain from about 10 wt% to about 75 wt% of 1,4:3,6-dianhydrohexitol. Properties of the polyurethane may also be controlled by use of one or more other polyols not containing 1,4:3,6-dianhydrohexitol in combination with the 1,4:3,6-dianhydrohexitol containing polyol.

According to at least one embodiment, other renewable source compounds may be used in place of or in addition to the 1,4:3,6:-dianhydrohexitol. For example, the nonionic polyol a1) may be a polyester polyol and comprise (or may be derived from) renewable versions of polyacids and polyols such as unsaturated fatty acids, $C_{36}$ and $C_{54}$ dimer and trimer products and furan derivatives such as 2,5-furandicarboxylic acid. These and other exemplary polyols and polyacids or cyclic esters available from renewable resources are shown below.

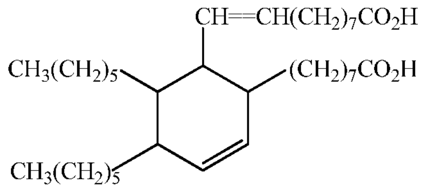

**Dimer Fatty Acids**

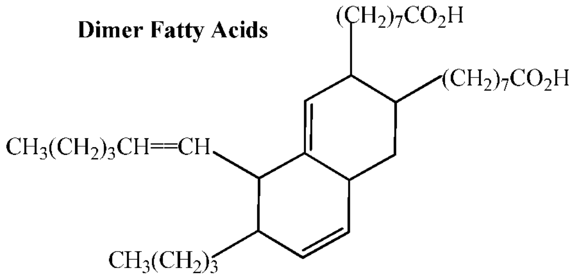

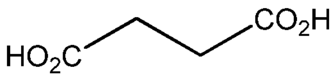

Succinic Acid

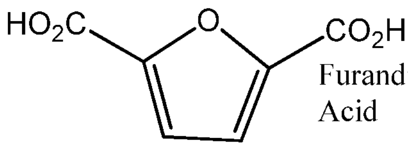

Furandicarboxylic Acid

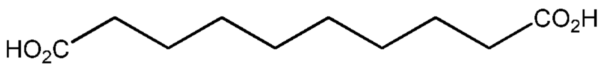

Sebacic Acid

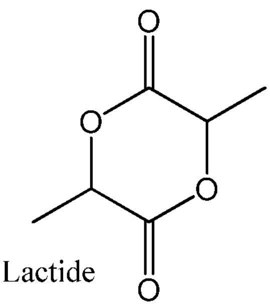

Lactide

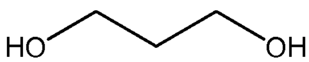

1,3-propanediol

The at least one polyol component a) also comprises a2) at least one polyol bearing at least one ionic or potentially ionic group, in particular comprising an acid group or salt thereof. The acid group may be selected from a carboxy group (-$CO_2H$), a sulfonic group (-$SO_3H$), sulfonyl group (-$SO_2H$), a phosphoryl group (-$PO_3H_2$) and a phosphonyl group (-$PO_2H$). According to at least one embodiment, the acid group of the at least one polyol a2) is selected from a carboxy group, a sulfonic group or a sulfonyl group and is preferably a carboxyl group. Examples of acid group-containing polyols a2) include, but are not limited to, 2-carboxy 1,3-propane diol, 2-sulfo 1,3-propane diol, 2-methyl-2-carboxy hexane diol, 3-methyl-3-carboxy hexane diol. 4-methyl-4-carboxy hexane diol. 2-ethyl-2-carboxy 1,3-propane diol. 2-ethyl-2-carboxy butane diol, dimethylolpropionic acid, dimethylolbutanoic acid, 2-sulfo-1,4-butanediol, 2,5-dimethyl-3-sulfo-2,5-hexanediol, 2-aminoethanesulfonic acid, N-(1,1-dimethyl-2-hydroxyethyl)-3-amino-2-hydroxypropanesulfonic acid, 2-aminoethylaminoethanesulfonic acid and salts of the above.

The at least one polyol component a) further comprises a3) comprising at least one ethylenically unsaturated a3.1) monoalcohol and/or a3.2) polyol. Said monoalcohol a3.1) or polyol a3.2) bears from 2 to 5 ethylenically unsaturated groups, which are (meth)acrylate groups, per molecule. Preferably, the OH functionality of said polyol a3.2) may vary from 2 to 5 and preferably said ethylenically unsaturated polyol a3.2) is an ethylenically unsaturated diol.

The monoalcohols as defined according to a3.1) do introduce terminal ethylenic unsaturations, while diols introduce lateral unsaturations and higher functional polyols create branched structures with unsaturation in both the polyurethane main chain backbone (lateral or side unsaturation) and in grafted branches (lateral unsaturation but on grafted polyurethane branches). According to a specific embodiment, said polyol component a3) is a mixture of monoalcohols a3.1) and of polyols a3.2). According to at least one embodiment, the at least one ethylenically unsaturated monoalcohol or polyol comprises a monool or diol. Examples of ethylenically unsaturated monoalcohol a3.1) or polyols a3.2) include, but are not limited to, polyester (meth)acrylates, epoxy (meth)acrylates, polyether (meth)acrylates, polyurethane (meth)acrylates and other hydroxyl group-containing (meth)acrylates. As examples of hydroxyl-containing (meth)acrylates, we may cite without limitation as monools : a multifunctional (meth)acrylate having a free hydroxyl, like trimethylol propane di(meth)acrylate which may be alkoxylated, ditrimethylol propane tri(meth)acrylate which may be alkoxylated, pentaerythritol penta(meth)acrylate which may be alkoxylated. As examples of hydroxyl-containing polyol (meth)acrylates suitable for a3.2), we may cite : ditrimethylol di(meth)acrylate (diol), pentaerythritol di(meth)acrylate (diol), dipentaerythritol tetra(meth)acrylate (diol), dipentaerythritol di (meth)acrylate (tetrol), dipentaerythritol tri-(meth)acrylate (triol), all of the above being potentially alkoxylated. Alkoxylated units may be ethoxy and/or propoxy and/or tetramethylenoxy with at least one alkoxy unit by OH, preferably from 1 to 10 and more preferably from 1 to 6 alkoxy units by OH.

In at least one embodiment, the at least one polyol a) component comprises carbon atoms from renewable resources. For example, the renewably resourced carbon atoms may be provided by the non-ionic polyol a1), the acid group-containing polyol a2) and/or the ethylenically unsaturated monoalcohol or polyol a3). In at least one embodiment, the renewably resourced carbon atoms may be provided by the non-ionic polyol a1) and/or the ethylenically unsaturated monoalcohol or polyol a3). According to at least one preferred embodiment, the non-ionic polyol a1) comprises renewably resourced carbon atoms.

The use of carbon-based starting materials of natural and renewable origin can be detected by virtue of the carbon atoms participating in the composition of the final product. This is because, unlike substances resulting from fossil materials, substances composed of renewable starting materials comprise $^{14}C$. All carbon samples drawn from living organisms (animals or plants) are in fact a mixture of 3 isotopes : $^{12}C$ (representing ~98.892%), $^{13}C$ (~1.108%) and $^{14}C$ (~$1.2\times10^{-10}$%). The $^{14}C/^{12}C$ ratio of living tissues is identical to that of the atmosphere. In the environment, $^{14}C$ exists in two predominant forms : in inorganic form, e.g., carbon dioxide gas ($CO_2$) and in organic form, e.g., carbon incorporated in organic molecules. In a living organism, the $^{14}C/^{12}C$ ratio is kept constant metabolically as the carbon is continually exchanged with the environment. As the proportion of $^{14}C$ is substantially constant in the atmosphere, it is the same in the organism, as long as it is living, since it absorbs the $^{14}C$ like it absorbs the $^{12}C$. The mean $^{14}C/^{12}C$ ratio is equal to $1.2\times10^{-12}$. $^{12}C$ is stable, that is to say that the number of $^{12}C$ atoms in a given sample is constant over time. $^{14}C$ for its part is radioactive and each gram of carbon of a living being comprises sufficient $^{14}C$ isotope to give 13.6 disintegrations per minute. The half-life (or period) $T_{1/2}$, related to the disintegration constant of $^{14}C$, is 5730 years. Due to this period of time, the $^{14}C$ content is regarded as constant in practice from the extraction of the naturally renewable starting materials to the manufacture of the final product.

In at least one embodiment, the at least one polyol component a) of the present disclosure has a content by weight of $^{14}C$ such that the $^{14}C/^{12}C$ ratio is greater than $0.1\times10^{-12}$. According to at least one embodiment, the at least one polyol component a) has a content by weight of $^{14}C$ such that the $^{14}C/^{12}C$ ratio is greater than and preferably greater than $0.2\times10^{-12}$, greater than $0.4\times10^{-12}$, greater than $0.6\times10^{-12}$, greater than $0.8\times10^{-12}$ or greater than $1.0\times10^{-12}$.

Currently, there exist at least two different techniques for measuring the $^{14}C$ content of a sample :

- by liquid scintillation spectrometry or
- by mass spectrometry in which the sample is reduced to graphite or to gaseous $CO_2$ and analyzed in a mass

spectrometer. This technique uses an accelerator and a mass spectrometer to separate the $^{14}C$ ions from the $^{12}C$ ions and to thus determine the ratio of the two isotopes.

All these methods for measuring the $^{14}C$ content of substances are clearly described in the standards ASTM D 6866 (in particular D6866-06) and in the standards ASTM D 7026 (in particular 7026-04). The measurement method preferably used is the mass spectrometry described in the standard ASTM D 6866-06 (accelerator mass spectroscopy).

Examples of epoxy (meth)acrylates as suitable monoalcohol or polyol a3) include the reaction products of acrylic or methacrylic acid or mixtures thereof with glycidyl ethers or esters. The glycidyl ethers or esters can have aliphatic, cycloaliphatic or aromatic structures and contain from two up to about six epoxy functional groups. Di-epoxy functional materials are preferred. Glycidyl ethers can be prepared from a hydroxyl functional precursor and an epoxy compound such as epichlorohydrin. Many of the hydroxyl functional components listed in the section above are suitable for preparation of aliphatic glycidyl ethers. Specific examples of precursors for aliphatic glycidyl ethers include : 1,4-butanediol, 2,2-dimethyl-1,3-propanediol, 1,6-hexanediol, 1,4- and 1,6-dimethylolcylcohexane, poly(ethylene glycol), polypropylene glycol), poly(tetramethylene glycol), trimethylolpropane, pentaerythritol, glycerol and sorbitol. Specific examples of precursors for aromatic glycidyl ethers include : bisphenol A, bisphenol F and resorcinol.

Suitable epoxy (meth)acrylates can also include the reaction products of acrylic or methacrylic acid or mixtures thereof with epoxidized derivatives of natural oils and their component fatty acids such as soybean, linseed, castor, rapeseed, safflower, olive, tall and others which will be known to those skilled in the art.

Examples of suitable polyether (meth)acrylates as suitable monoalcohol or polyol a3) include the condensation reaction products of acrylic or methacrylic acid or mixtures thereof with polyether polyols. Suitable polyether polyols can be linear or branched substances containing ether bonds and terminal hydroxyl groups. Polyether polyols can be prepared by ring opening polymerization of cyclic ethers such as tetrahydrofuran or alkylene oxides with an initiator molecule. Suitable initiator molecules include water, alcohols (including polyols) and amines. Examples of suitable amines include : ethylene diamine, 4,4'-diaminodiphenylmethane, diethylene triamine and hydroxyl amines such as ethanol amine and diethanol amine. Examples of suitable alkylene oxides include : ethylene oxide, propylene oxide, butylenes oxides, epichlorohydrin and glycidol. The polyether (meth)acrylates can be used individually or in combination.

Examples of polyurethane (meth)acrylates as suitable monoalcohol or polyol a3) include the polyaddition products of the di- or polyisocyanates described below with isocyanate reactive ethylenically unsaturated components as described in the sections above as polyester-, epoxy- or polyether (meth)acrylates or immediately below as monomeric hydroxyl (meth)acrylates.

Examples of monomeric hydroxyl group-containing (meth)acrylates as suitable monoalcohol or polyol a3) are acrylic, methacrylic or mixed esters with simple triols, tetrols or polyols, where the esterification process is carried out such that residual hydroxyl groups remain in the final product. Examples include (meth)acrylate esters of : 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1 ,3-propanediol, diethylene glycol, triethylene glycol, dipropylene glycol, tripropylene glycol, 1,4- and 1,6-dimethylolcylcohexane, glycerol, trimethylolpropane, trimethylolethane, pentaerythritol, di-glycerol, di-trimethyolpropane, di-pentaerytritol and sorbitol. The monomeric hydroxyl (meth)acrylates can be used individually or in mixtures.

In accordance with at least one embodiment, the polyisocyanate b) comprises at least two isocyanate functional groups. In at least one embodiment, the polyisocyanate b) may comprise a diisocyanate having two isocyanate functional groups, such as, an aliphatic diisocyanate (e.g., isophorone diisocyanate). In other embodiments, the polyisocyanate may comprise a plurality of isocyanate groups, such as three or four or more isocyanate groups.

Non-limiting examples of compounds that may comprise the polyisocyanate include : di- or polyisocyanates such as aliphatic, aromatic and cycloaliphatic structures with at least two isocyanate functional groups per molecule. Examples of suitable polyisocyanate b) components include : isophorone diisocyanate, hexamethylene diisocyanate, 2,3,3-trimethyl hexamethylene diisocyanate, 4,4'-dicylcohexylmethane diisocyanate, 1,5-naphthalene diisocyanate, 2,4- or 2,6-toluene diisocyanate and their isomeric mixtures, 4,4'-diphenylmethane diisocyanate and their respective dimeric or trimeric derivatives and mixtures thereof. The said polyisocyanates may also be modified by allophanate groups.

Polyisocyanates formed by creation of isocyanurate (especially in diisocyanate trimers) or biuret structures (especially in diisocyanate dimers) are also suitable as are mixtures of isocyanates. Polyisocyanate b) with a functionality of 3 may be an isocyanurate triisocyanate, bearing a triisocyanurate ring, which may provide high thermal stability performance. Polyisocyanates based on renewable resources such as those described, for example at page 2, line 12 to page 6, line 10 in WO 2011/098272, may also be used in accordance with the present disclosure.

In accordance with at least one embodiment, a pre-polymer may be formed by reacting the polyol component a) with the polyisocyanate b) described above. In at least one embodiment, the number of isocyanate (NCO) groups of the at least one polyisocyanate b) is in excess with respect to the reacting number of hydroxyl (OH) groups of the at least one polyol component a).

In at least one embodiment, the acid group of polyol a2) can be neutralized to the salt form before or during

dispersion by addition of a base. Suitable bases include inorganic hydroxides or carbonates and amines and combinations. In at least one preferred embodiment, the acid group of polyol a2) is neutralized with a tertiary amine.

The polyols suitable for the present invention are those used to form polyurethane dispersions in accordance with the methods disclosed in provisional U.S. Patent Application No. 61/907,434 titled "Solvent-free aqueous polyurethane dispersions and methods of producing solvent-free aqueous polyurethane dispersions", filed on November 22, 2013 and provisional U.S. Patent Application No. 61/986,165 titled "Solvent-free aqueous polyurethane dispersions and methods of producing solvent-free aqueous polyurethane dispersions", filed concurrently herewith.

The polyurethane dispersion may also be formed using additional components.

According to at least one embodiment, the at least one polyol a) and the at least one polyisocyanate b) may be reacted in the presence of a curable or reactive diluent, which means an ethylenically unsaturated diluent which is able to co-react during the cure with said ethylenically unsaturated polyurethane. The curable or reactive diluent is at least one ethylenically unsaturated monomer or oligomer, preferably monomer, which is compatible (non demixing) with said curable polyurethane. The curable diluent, for example, may comprise materials with two or more ethylenically unsaturated groups, such as, for example, (meth)acrylate groups. The curable diluents, which can be monomeric or oligomeric, can be used individually or in combination. Suitable monomeric examples include the (meth)acrylate esters of 1,2-ethanediol, 1,2-propanediol, 1,3-propanediol, 1,4-butanediol, 1,3-butanediaol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, tetraethylene glycol, dipropylene glycol, tripropylene glycol, 1,4- and 1,6-dimethylolcylcohexane, glycerol, trimethylolpropane, trimethylolethane, pentaerythritol, di-glycerol, di-trimethyolpropane, di-pentaerytritol, sorbitol and alkoxylated derivatives of the above. Many such materials are available from Sartomer as "SR" products. The reactive diluent helps to lower the minimum film formation temperature (MFFT) of the system, promote particle coalescence and also contributes to cured coating properties. So, according to a specific option, the polyurethane polymer aqueous dispersion of the invention further comprises a curable ethylenically unsaturated monomer acting as a curable diluent and which diluent is present in the reaction of step i) as defined above, related with the pre-polymer formation. In fact, said diluent is used as a reaction diluent in step i) and as a reactive diluent during the cure of the curable polyurethane polymer.

Oligomeric curable diluents include the polyester-, polyether- or urethane (meth)acrylates as described above, except that when used as a reactive diluent the hydroxyl group is fully (meth)acrylated. Many such products are available from Sartomer as "CN" products.

The polyurethane pre-polymer is subjected to a chain extension reaction to form the polyurethane polymer. The chain extension further increases the molecular weight of the polyurethane and/or vary or adjust the properties of the final said curable polyurethane. For example, the chain extender c) may be selected to alter/adjust the hardness, weatherability, flexibility or adhesiveness. The chain extenders c) bear at least two isocyanate-reactive groups which are OH or amine and c) may be selected from polyols and polyamines, such as, for example, diols and diamines, preferably diamines. In at least one preferred embodiment, the chain extender c) is selected from polyamines and more preferably from diamines. Following chain extension, the polyurethane polymer may have a number average molecular weight Mn ranging from about 1500 to about 60000 Daltons, such as, for example, from about 2000 to about 50000 Daltons. The molecular weights Mn are determined by gel permeation chromatography (GPC) in THF using polystyrene calibration standards. The molecular weight and polydispersity are thus determined by conventional gel permeation chromatography (GPC). A small sample is dissolved in tetrahydrofuran (THF) and injected into a liquid chromatograph (Agilent 1100 Series) equipped with HP PLGel® GPC columns (5 um, 100A, 250x 4.6 mm; 3 um MiniMix-E, 250x4.6 mm and 5 um MiniMix-D, 250x4.6 mm). The components of the sample are separated by the GPC columns based on their molecular sizes in solution. The components are detected by an Agilent 1047A® refractive index detector and recorded by Agilent HPLC Chemstation® and Polymer Laboratories GPC software. Polystyrene standards of known molecular weight and narrow dispersity are used to generate a calibration curve.

The chain extender c) may comprise two or more functional groups reactive with the isocyanate terminal groups of the polyurethane pre-polymer. In at least one embodiment, the chain extender c) comprises two isocyanate-reactive functional groups and functions to extend the polyurethane. In other embodiments, the chain extender may comprise three or more functional groups and function to both extend the polyurethane chain and increase the branching of the chains without any crosslinking. In at least one embodiment, a mixture of chain extenders comprising two functional groups and three or more functional groups may be used. In case the global number average functionality (over components a + b + chain extender c) is higher than 2, then branching occurs. The curable polyurethane of the present invention may have a linear or branched chain structure without any crosslinked structure present in the said curable polyurethane either before or after its aqueous dispersion and chain extension. In case the global average functionality is higher than 2, those skilled in the art know how to prevent any crosslinking reaction. Generally, the addition of one reactant to the other is made progressively and the proportions of the reactants (ratio of NCO groups to NCO-reactive groups) and the conversion of these groups is so controlled in order to fulfill the Macosko-Miller relationship as disclosed in Macromomecules, vol. 9, pp199-221 (1976). This relationship is relating the critical ratio $r_c$, or limit conversion $x_g$ (limit

of gelification by crosslinking when fixing other parameters) to the average number functionality $f_A$ and $f_B$ of respectively the main reactants A and B with A being such as polyol a) and B such as polyisocyanate b) for the pre-polymer reaction and A being such as the chain extender and B being such as the NCO pre-polymer for the polyurethane extension reaction. Macosko-Miller relationship to be fulfilled is as follows :

$$r_c = x_g^2\ 1[\ (f_B-1)*(\ f_A-1)$$

In at least one embodiment, the reaction mixture for forming the polyurethane dispersion or pre-polymer may also comprise a catalyst and/or other additives, such as, for example, inhibitors, fillers, stabilizers, photoinitiators, pigments, etc. External surfactants are not necessary and could be used only optionally.

According to at least one embodiment of the present disclosure, the polyurethane dispersion and the related nail polish formulation are radiation curable or peroxide curable. In at least one embodiment, the polyurethane dispersion and the related nail polish formulation may be cured by exposure to actinic radiation. According to at least one embodiment, the polyurethane dispersion and the related nail polish formulation are cured by exposure to ultraviolet light.

The polyurethane dispersions of the present disclosure may also be used in coatings, in particular to coat objects, such as for example, wood, metal, plastic, ceramic, composite objects, glass, fibers, textiles, leather, stone, concrete and other materials. The object may be coated with the polyurethane dispersion, which is subsequently cured.

The polyurethane dispersions of the present disclosure may be used to form coatings that provide protection against mechanical, chemical and/or environmental effects. The polyurethane dispersions may also be used as adhesives, sealants, surface modifiers, surface coatings and inks.

More particularly, according to the present invention, said polyurethanes aqueous dispersions are used in nail polishes.

In at least one embodiment, the polyurethane dispersions of the present disclosure may be used to form a radiation curable or peroxide curable composition, more particularly a nail polish composition or formulation.

The particular interest of said polyurethane dispersions of the present disclosure in their application is that they provide surface texture or haptic effects.

The polyurethane coatings formed from polyurethane dispersions of the present disclosure may be used to provide scratch, abrasion and wear resistance ; UV protection ; surface appearance, such as a glossy or flat appearance ; chemical and stain resistance ; hydrolytic resistance ; anti-microbial activity ; adhesion ; haptic effects such as soft touch ; easy cleaning and anti-fingerprint. The properties of the resultant polyurethane coatings may be controlled by varying the amounts of the components present within the polyurethane dispersions described above.

The present disclosure particularly relates to nail varnish or nail polish formulations and the use of the aqueous polyurethane dispersions disclosed herein as nail polish formulations. The aqueous polyurethane dispersions described above are specifically formulated for application to the nails. The actinic radiation curable polyurethanes may be cured upon exposure to sunlight or UV/LED lamps to create a hard and glossy/shiny coating, in particular a nail coating.

In at least one embodiment, the nail polish composition comprising an aqueous polyurethane dispersion, a photoinitiator and optionally a leveling agent and/or a thickener. A leveling agent may be included to aid in the formation of a smooth and homogeneous surface of the coating. The leveling agent may be selected from any known leveling agent for use in nail polish formulations, such as, for example, BYK®-346 (available from BYK-CHEMIE GMBH).

Thickeners may be added to the nail polish formulation to increase the viscosity of the formulation and/or to control the flow properties of the formulation. Non-limiting examples of thickeners include Acrysol® RM-825, Aculyn® 33 and Aculyn® 44 (available from Dow Chemical Co.).

In at least one embodiment, the nail polish formulation may also comprise a coloring agent, such as a pigment, dyes or other colorants and may also comprise solid particulates or granulates to be applied as part of the nail coating to provide a three dimensional and/or non-smooth and/or flecked surface. The nail polish formulation may further comprise fillers, surfactants, stabilizers, fragrances and/or preservatives.

In accordance with at least one embodiment, the nail polish formulation has a viscosity ranging from about 250 mPa.s (250 cP) to about 4000 mPa.s (4000 cP) or from about 500 mPa.s (500 cP) to about 3000 mPa.s (3000 cP) or from about 1000 mPa.s (1000 cP) to about 2000 mPa.s (2000 cP) at room temperature. "Room temperature" here means 25°C. The viscosity is determined using Brookfield method at 100 rpm.

According to at least one embodiment, the nail polish formulation has a solids level (content) ranging from about 10% to about 40% or from about 20% to 30% or to about 25% w/w.

According to at least one embodiment, the nail polish formulation is free or substantially free of non-reactive solvents. More preferably, the content of non-reactive solvent is 0.

The following examples are given for illustrating the present invention and its performances and does not at all limit the covering of the invention.

## EXAMPLES

Example 1

A polyester polyol was made using the following procedure : 1,3-propanediol (177 g, from DuPont Tate&Lyle), isosorbide (681 g, from Roquette), succinic acid (551 g, from BioAmbe), which were all derived from renewable sources along with toluene (388 g) and 70% methanesulfonic acid in water (17 g) were charged to a reaction vessel with fitted with a side arm for collection of evolved water. The mixture was heated to 112°C for 21 hours at which time production of water had substantially ceased. After removal of the toluene by vacuum distillation, the final polyester polyol product was a viscous liquid with hydroxyl number of 200 mg KOH/g.

All cited OH values are determined by Radiometer TitraLab® TM865 Autotitrator. The detailed method is as follows :

A 4-5 g sample is dissolved in 25 ml acetonitrile (CH3CN), then 25 ml p-toluenesulfonyl isocyanate (TSI) reagent is added volumetrically and stirred for 10 minutes. The sample is then titrated with 0.25 M concentration tetrabutylammonium hydroxide. The results are reported in mg KOH/g by the autotitrator.

Example 2

A polyester polyol was prepared from isosorbide (98 g) and d,l-lactide (240 g) by heating to 120°C for several hours in the presence of stannous octoate catalyst. The final polyester polyol product was a viscous liquid with hydroxyl number of 211 mg KOH/g.

Example 3 - Preparation of a UV-curable Polyurethane Dispersion (UV-PUD)

A reaction vessel suitable for polyurethane preparation was charged with ditrimethylol propane triacrylate with OH value = 163 mg KOH/g (206 g), the polyester polyol of Example 1 (388 g), dimethylolpropionic acid (113 g), methyl hydroquinone MeHQ (4.8 g), dibutyltin dilaurate (3.2 g) and ethoxylated trimethylolpropane triacrylate (Sartomer SR454, 1603 g) and the mixture was heated to 50°C. Isophorone diisocyanate (888 g) was added over 30 minutes while increasing the temperature to 70°C. The reaction was held at 70°C until the % NCO by titration was constant at which point the temperature was lowered to 55°C. Triethylamine (85 g) was added followed by, with vigorous agitation, de-ionized water (4577 g). After 5 minutes, ethylenediamine (92 gm) dissolved in de-ionized water (93 g) was added and agitation was continued for another 2 hours. After filtration through a 100 micron filter bag, the final dispersion had the following properties : w/w % solids = 42.0, viscosity at 25°C = 6.1 mPa.s (6.1 cP), average particle size = 114 nm and pH = 7.23.

Example 4 - Preparation of a UV-curable Polyurethane Dispersion (UV-PUD)

A reaction vessel suitable for polyurethane preparation was charged with ditrimethylol propane triacrylate with OH value = 163 mg KOH/g (130 g), the polyester polyol of Example 1 (101 g), poly(1,3-propanediol) (product of DuPont, 1000MW, 200 g) dimethylolpropionic acid (78 g), MeHQ (3.3 g), dibutyltin dilaurate (2.2 g) and ethoxylated trimethylolpropane triacrylate (Sartomer SR454, 1110 g) and the mixture was heated to 50°C. Isophorone diisocyanate (590 g) was added over 30 minutes while increasing the temperature to 70°C. The reaction was held at 70°C until the % NCO by titration was constant at which point the temperature was lowered to 55°C. Triethylamine (59 g) was added followed by, with vigorous agitation, de-ionized water (3135 g). After 5 minutes, ethylenediamine (58 g) dissolved in de-ionized water (64 g) was added and agitation was continued for another 2 hours. After filtration through a 100 micron filter bag, the final dispersion had the following properties : w/w % solids = 41.3, viscosity at 25°C = 10.2 mPa.s (10.2 cP), average particle size = 126 nm and pH = 7.2.

Example 5 - Preparation of a UV-curable Polyurethane Dispersion (UV-PUD)

The procedure of Example 4 was followed but the following recipe was used : dimethylolpropanetriacrylate with OH value = 163 mg KOH/g (124 g), the polyester polyol of Example 1 (210 g), poly(1,3-propanediol) (product of DuPont, 1000MW, 140 g), dimethylolpropionic acid (84 g), MeHQ (3.6 g), dibutyltin dilaurate (2.4 g) and ethoxylated trimethylolpropane triacrylate (Sartomer SR454, 1195 g), isophorone diisocyanate (631 g), triethylamine (63 g), de-ionized water (3408 g) and ethylene diamine + water (68 g + 70 g). After filtration through a 100 micron filter bag, the final dispersion had the following properties : w/w % solids = 41.1, viscosity at 25°C = 10.2 mPa.s (10.2 cP), average particle size = 176 nm and pH = 7.25.

Example 6 - Preparation of a UV-curable Polyurethane Dispersion (UV-PUD)

A reaction vessel suitable for polyurethane preparation was charged with dimethylolpropionic acid (85 g), MeHQ (2.8 g), dibutyltin dilaurate (1.9 g), ethoxylated trimethylolpropane triacrylate (Sartomer SR454, 1204 g) and isophorone diisocyanate (572 g) and the mixture was heated to 70°C until the %NCO content by titration dropped to about 7.9%. Dimethylolpropanetriacrylate with OH value = 163 mg KOH/g (98 g), the polyester polyol of Example 2 (145 g), poly(1,3-propanediol) (product of DuPont, 1000MW, 298 g) MeHQ (3.6 g) and an additional 1.9 g of dibutyltin dilaurate were then added and the temperature maintained at 70°C until a constant % NCO content was achieved. The temperature was reduced to 55°C and triethylamine (64 g) was added followed by, with vigorous agitation, de-ionized water (3359 g). After 5 minutes, ethylenediamine (49 g) dissolved in de-ionized water (120 g) was added and agitation continued for another two hours. After filtration through a 100 micron filter bag, the final dispersion had the following properties : w/w % solids = 40.6, viscosity at 25°C = 12.6 mPa.s (12.6 cP), average particle size = 118 nm and pH = 7.79.

Example 7 - Use of the prepared curable aqueous polyurethane dispersions in nail polish formulation

97.73 wt parts of the polyurethane dispersion of Example 4, which has been diluted with water to 35% solids, is mixed with 0.17 wt part of a leveling agent (BYK®-346, available from BYK-CHEMIE GMBH), 1.74 wt part of a photoinitiator (Irgacure 500) and about 0.35 wt part of a thickener Acrysol® RM825 to form a nail polish formulation (100 wt parts).

The formulation is coated on aluminum and cured under UV energy of 1 J/cm$^2$. The 60° gloss is about 116.

The formulation is applied to acrylic artificial nails using a brush applicator. Once the water evaporates, a homogeneous film is formed without brush marks. After curing under UV energy of 1 J/cm$^2$, the formulation is expected to produce a smooth, homogeneous, shiny film. The adhesion of the nail polish formulation on the acrylic artificial nails is excellent and unable to be peeled or cracked off using finger nails.

## Claims

**1.** A nail polish formulation comprising :

- a curable aqueous polyurethane dispersion ;
- a photoinitiator ;
- optionally a leveling agent and
- optionally a thickener

wherein the formulation is free of non-reactive solvents and wherein said curable aqueous polyurethane dispersion is formed by the reaction of a) at least one polyol component comprising a1) a non-ionic polyol, a2) at least one polyol bearing at least one ionic or potentially ionic group comprising an acid group or salt thereof and a3) at least one ethylenically unsaturated monoalcohol or polyol, with b) at least one polyisocyanate, wherein the number of isocyanate (NCO) groups of the at least one polyisocyanate b) is in excess with respect to the number of hydroxyl (OH) groups of the at least one polyol component a), wherein said at least one polyol component a1) comprises carbon atoms from renewable resources, said acid group of polyol a2) being at least partly neutralized and said reaction being followed in a second step by an extension reaction with an isocyanate-reactive chain extender c) and wherein said at least one ethylenically unsaturated monoalcohol or polyol a3) comprises at least one ethylenically unsaturated monoalcohol a3.1) and/or polyol a3.2), with said monoalcohol a3.1) or polyol a3.2) bearing from 2 to 5 ethylenically unsaturated groups per molecule the said ethylenically unsaturated groups being (meth)acrylate groups.

**2.** The nail polish formulation according to claim 1, wherein the at least one non-ionic polyol a1) comprises a polyol formed by reacting a 1,4:3,6-dianhydrohexitol in a polycondensation polymerization reaction with at least one diacid or higher functional carboxylic acid, by reacting a 1,4:3,6-dianhydrohexitol with a hydroxyl functional monocarboxylic acid or by reacting a 1,4:3,6-dianhydrohexitol with caprolactone.

**3.** The nail polish formulation according to claim 2, wherein the 1,4:3,6-dianhydrohexitol is selected from the group consisting of isosorbide, isomannide and isoidide.

**4.** The nail polish formulation according to claim 3, wherein the 1,4:3,6-dianhydrohexitol is isosorbide.

5. The nail polish formulation according to claim 1, wherein the at least one non-ionic polyol a1) comprises a polyol formed by reacting a 1,4:3,6-dianhydrohexitol in a polycondensation polymerization reaction with at least one diol or polyol and at least one diacid or higher functional carboxylic acid, wherein the diol or polyol is selected from the group consisting of 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, $C_{36}$-dimer diol, hydroxypivaloyl hydroxyl pivalate and ethoxylated and/or propoxylated derivatives thereof.

6. The nail polish formulation according to claim 5, wherein the said at least one diacid or higher functional carboxylic acid is selected from the group consisting of malonic acid, succinic acid, maleic acid, fumaric acid, itaconic acid, glutaric acid, citric acid, adipic acid, pimelic acid, sebacic acid, dodecanedioic acid, phthalic acid, isophthalic acid, terephthalic acid, naphthalene dicarboxylic acid, $C_{36}$-dimer fatty acids, $C_{54}$-trimer fatty acids (triacid) trimellitic acid, pyromellitic acid and anhydrides thereof.

7. The nail polish formulation according to claim 1, wherein said non-ionic polyol a1) is formed using a 1,4:3,6-dianhydrohexitol as a hydroxyl functional initiator molecule in a ring opening polymerization of lactide.

8. The nail polish formulation according to any one of claims 1 to 7, wherein said monoalcohol or polyol a3) is an isocyanate-reactive ethylenically unsaturated monoalcohol or polyol selected from the group consisting of polyester (meth)acrylates, epoxy (meth)acrylates, polyether (meth)acrylates, polyurethane (meth)acrylates and hydroxyl group-containing (meth)acrylates.

9. The nail polish formulation according to any one of claims 1 to 8, wherein said curable aqueous polyurethane dispersion and said nail polish formulation is a radiation- or peroxide-curable dispersion.

10. The nail polish formulation according to any one of claims 1 to 9, wherein the at least one polyol component a) has a content by weight of $^{14}C$ such that the $^{14}C/^{12}C$ ratio is greater than $0.1\times10^{-12}$.

11. The nail polish formulation according to any one of claims 1 to 10, further comprising at least one coloring agent.

12. The nail polish formulation according to any one of claims 1 to 11, wherein the said nail polish formulation is UV curable.

13. The nail polish formulation according to any one of claims 1 to 12, wherein the formulation has a solids level of 20% to 30% w/w and a viscosity of 500 mPa.s (500 cP) to 3000 mPa.s (3000 cP) at room temperature.

14. The nail polish formulation, according to any one of claims 1 to 13, wherein said chain extender c) bears two isocyanate-reactive groups from hydroxyl or amine and preferably amine and more preferably chain extender c) is a diamine.

15. Use of the nail polish formulation according to any one of claims 1 to 14, for coating nails.

16. Use of the curable aqueous polyurethane dispersion as defined in any one of claims 1 to 10, in a nail polish formulation.

17. Nail coating, wherein it results from the cure of a nail polish formulation as defined in any one of claims 1 to 14 or wherein it results from a use as defined in one of claims 15 or 16.

**Patentansprüche**

1. Nagellackformulierung, umfassend:

- eine härtbare wässrige Polyurethandispersion,
- einen Photoinitiator,
- gegebenenfalls ein Verlaufsmittel und
- gegebenenfalls einen Verdicker,

wobei die Formulierung frei von nichtreaktiven Lösungsmitteln ist und wobei die härtbare wässrige Polyurethandispersion durch die Umsetzung von a) mindestens einer Polyolkomponente, die a1) ein nichtionisches Polyol, a2) mindestens ein Polyol mit mindestens einer ionischen oder potentiell ionischen Gruppe, die eine Säuregruppe oder ein Salz davon umfasst, und a3) mindestens einen ethylenisch ungesättigten Monoalkohol oder mindestens ein ethylenisch ungesättigtes Polyol umfasst, mit b) mindestens einem Polyisocyanat gebildet wird, wobei die Zahl von Isocyanat(NCO)-Gruppen des mindestens einen Polyisocyanats b) im Überschuss in Bezug auf die Zahl von Hydroxyl(OH)-Gruppen der mindestens einen Polyolkomponente a) vorliegt,
wobei die mindestens eine Polyolkomponente a1) Kohlenstoffatome aus erneuerbaren Quellen umfasst, wobei die Säuregruppe von Polyol a2) mindestens teilweise neutralisiert ist und wobei auf die Umsetzung in einem zweiten Schritt eine Verlängerungsreaktion mit einem isocyanatreaktiven Kettenverlängerungsmittel c) folgt und
wobei der mindestens eine ethylenisch ungesättigte Monoalkohol bzw. das mindestens eine ethylenisch ungesättigte Polyol a3) mindestens einen ethylenisch ungesättigten Monoalkohol a3.1) und/oder mindestens ein ethylenisch ungesättigtes Polyol a3.2) umfasst, wobei der Monoalkohol a3.1) bzw. das Polyol a3.2) 2 bis 5 ethylenisch ungesättigte Gruppen pro Molekül trägt, wobei es sich bei den ethylenisch ungesättigten Gruppen um (Meth)acrylat-Gruppen handelt.

2. Nagellackformulierung nach Anspruch 1, wobei das mindestens eine nichtionische Polyol a1) ein durch Umsetzung eines 1,4:3,6-Dianhydrohexitols in einer Polykondensationspolymerisationsreaktion mit mindestens einer Disäure oder höherfunktionellen Carbonsäure, durch Umsetzung eines 1,4:3,6-Dianhydrohexitols mit einer hydroxylfunktionellen Monocarbonsäure oder durch Umsetzung eines 1,4:3,6-Dianhydrohexitols mit Caprolacton gebildetes Polyol umfasst.

3. Nagellackformulierung nach Anspruch 2, wobei das 1,4:3,6-Dianhydrohexitol aus der Gruppe bestehend aus Isosorbid, Isomannid und Isoidid ausgewählt ist.

4. Nagellackformulierung nach Anspruch 3, wobei es sich bei dem 1,4:3,6-Dianhydrohexitol um Isosorbid handelt.

5. Nagellackformulierung nach Anspruch 1, wobei das mindestens eine nichtionische Polyol a1) ein durch Umsetzung eines 1,4:3,6-Dianhydrohexitols in einer Polykondensationspolymerisationsreaktion mit mindestens einem Diol oder Polyol und mindestens einer Disäure oder einer höherfunktionellen Carbonsäure gebildetes Polyol umfasst, wobei das Diol bzw. Polyol aus der Gruppe bestehend aus 1,2-Ethandiol, 1,2-Propandiol, 1,3-Propandiol, 1,4-Butandiol, 1,5-Pentandiol, 1,6-Hexandiol, 1,10-Decandiol, 1,12-Dodecandiol, 1,3-Butandiol, 2,2-Dimethyl-1,3-propandiol, 2-Methyl-1,3-propandiol, Diethylenglykol, Triethylenglykol, Dipropylenglykol, Tripropylenglykol, 1,4- und 1,6-Dimethylolcylcohexan, $C_{36}$-Dimerdiol, Hydroxypivaloylhydroxylpivalat und ethoxylierten und/oder propoxylierten Derivaten davon ausgewählt ist.

6. Nagellackformulierung nach Anspruch 5, wobei die mindestens eine Disäure oder höherfunktionelle Carbonsäure aus der Gruppe bestehend aus Malonsäure, Bernsteinsäure, Maleinsäure, Fumarsäure, Itaconsäure, Glutarsäure, Citronensäure, Adipinsäure, Pimelinsäure, Sebacinsäure, Dodecandisäure, Phthalsäure, Isophthalsäure, Terephthalsäure, Naphthalindicarbonsäure, $C_{36}$-Dimerfettsäuren, $C_{54}$-Trimerfettsäuren (Trisäure), Trimellitsäure, Pyromellitsäure und Anhydriden davon ausgewählt ist.

7. Nagellackformulierung nach Anspruch 1, wobei das nichtionische Polyol a1) durch Verwendung eines 1,4:3,6-Dianhydrohexitols als hydroxylfunktionelles Initiatormolekül in einer Ringöffnungspolymerisation von Lactid gebildet wird.

8. Nagellackformulierung nach einem der Ansprüche 1 bis 7, wobei es sich bei dem Monoalkohol oder Polyol a3) um einen isocyanatreaktiven ethylenisch ungesättigten Monoalkohol oder ein isocyanatreaktives ethylenisch ungesättigtes Polyol aus der Gruppe bestehend aus Polyester(meth)acrylaten, Epoxy(meth)acrylaten, Polyether(meth)acrylaten, Polyurethan(meth)acrylaten und hydroxylgruppenhaltigen (Meth)acrylaten handelt.

9. Nagellackformulierung nach einem der Ansprüche 1 bis 8, wobei es sich bei der härtbaren wässrigen Polyurethandispersion und der Nagellackformulierung um eine strahlungshärtbare oder peroxidhärtbare Dispersion handelt.

10. Nagellackformulierung nach einem der Ansprüche 1 bis 9, wobei die mindestens eine Polyolkomponente a) einen solchen Gewichtsgehalt an $^{14}C$ aufweist, dass das $^{14}C/^{12}C$-Verhältnis größer als $0{,}1 \times 10^{-12}$ ist.

11. Nagellackformulierung nach einem der Ansprüche 1 bis 10, die ferner mindestens ein Farbmittel umfasst.

12. Nagellackformulierung nach einem der Ansprüche 1 bis 11, wobei die Nagellackformulierung UV-härtbar ist.

13. Nagellackformulierung nach einem der Ansprüche 1 bis 12, wobei die Formulierung einen Feststoffgehalt von 20 bis 30 % w/w und eine Viskosität von 500 mPa.s (500 cP) bis 3000 mPa.s (3000 cP) bei Raumtemperatur aufweist.

14. Nagellackformulierung nach einem der Ansprüche 1 bis 13, wobei das Kettenverlängerungsmittel c) zwei isocyanatreaktive Gruppen von Hydroxyl und Amin und vorzugsweise Amin trägt und es sich weiter bevorzugt bei dem Kettenverlängerungsmittel c) um ein Diamin handelt.

15. Verwendung der Nagellackformulierung nach einem der Ansprüche 1 bis 14 zum Beschichten von Nägeln.

16. Verwendung der härtbaren wässrigen Polyurethandispersion gemäß einem der Ansprüche 1 bis 10 in einer Nagellackformulierung.

17. Nagelbeschichtung, wobei sie sich aus der Härtung einer Nagellackformulierung gemäß einem der Ansprüche 1 bis 14 ergibt oder wobei sie sich aus einer Verwendung gemäß einem der Ansprüche 15 oder 16 ergibt.

**Revendications**

1. Formulation de vernis à ongles comprenant :

   - une dispersion aqueuse durcissable de polyuréthane ;
   - un photoinitiateur ;
   - éventuellement un agent nivelant et
   - éventuellement un épaississant

   la formulation étant exempte de solvants non réactifs et ladite dispersion aqueuse durcissable de polyuréthane étant formée par la réaction de a) au moins un composant de polyol comprenant a1) un polyol non ionique, a2) au moins un polyol portant au moins un groupe ionique ou potentiellement ionique comprenant un groupe acide ou un sel correspondant et a3) au moins un monoalcool ou polyol éthyléniquement insaturé, avec b) au moins un polyisocyanate, le nombre de groupes isocyanate (NCO) de l'au moins un polyisocyanate b) étant en excès par rapport au nombre de groupes hydroxyle (OH) de l'au moins un composant de polyol a), ledit au moins un composant de polyol a1) comprenant des atomes de carbone provenant de ressources renouvelables, ledit groupe acide du polyol a2) étant au moins partiellement neutralisé et ladite réaction étant suivie dans une deuxième étape par une réaction d'extension avec un extenseur de chaîne c) réactif envers isocyanate et
   ledit au moins un monoalcool ou polyol éthyléniquement insaturé a3) comprenant au moins un monoalcool éthyléniquement insaturé a3.1) et/ou un polyol a3.2), ledit monoalcool a3.1) ou polyol a3.2) portant de 2 à 5 groupes éthyléniquement insaturés par molécule, lesdits groupes éthyléniquement insaturés étant des groupes (méth)acrylate.

2. Formulation de vernis à ongles selon la revendication 1, l'au moins un polyol non ionique a1) comprenant un polyol formé par la mise en réaction d'un 1,4:3,6-dianhydrohexitol dans une réaction de polymérisation par polycondensation avec au moins un diacide ou un acide carboxylique à fonctionnalité supérieure, par la mise en réaction d'un 1,4:3,6-dianhydrohexitol avec un acide monocarboxylique fonctionnalisé par hydroxyle ou par la mise en réaction d'un 1,4:3,6-dianhydrohexitol avec la caprolactone.

3. Formulation de vernis à ongles selon la revendication 2, le 1,4:3,6-dianhydrohexitol étant choisi dans le groupe constitué par l'isosorbide, l'isomannide et l'isoidide.

4. Formulation de vernis à ongles selon la revendication 3, le 1,4:3,6-dianhydrohexitol étant l'isosorbide.

5. Formulation de vernis à ongles selon la revendication 1, l'au moins un polyol non ionique a1) comprenant un polyol formé par la mise en réaction d'un 1,4:3,6-dianhydrohexitol dans une réaction de polymérisation par polycondensation avec au moins un diol ou polyol et au moins un diacide ou acide carboxylique à fonctionnalité supérieure, le diol ou le polyol étant choisi dans le groupe constitué par le 1,2-éthanediol, le 1,2-propanediol, le 1,3-propanediol, le 1,4-butanediol, le 1,5-pentanediol, le 1,6-hexanediol, le 1,10-décanediol, le 1,12-dodécanediol, le 1,3-butanediol, le 2,2-diméthyl-1,3-propanediol, le 2-méthyl-1,3-propanediol, le diéthylèneglycol, le triéthylèneglycol, le dipropylèneglycol, le tripropylèneglycol, le 1,4- et le 1,6-diméthylolcyclohexane, le $C_{36}$-dimère diol, l'hydroxypivaloylhydroxylpivalate et des dérivés éthoxylés et/ou propoxylés correspondants.

**6.** Formulation de vernis à ongles selon la revendication 5, ledit au moins un diacide ou acide carboxylique à fonctionnalité supérieure étant choisi dans le groupe constitué par l'acide malonique, l'acide succinique, l'acide maléique, l'acide fumarique, l'acide itaconique, l'acide glutarique, l'acide citrique, l'acide adipique, l'acide pimélique, l'acide sébacique, l'acide dodécanedioïque, l'acide phtalique, l'acide isophtalique, l'acide téréphtalique, l'acide naphtalènedicarboxylique, les acides gras $C_{36}$-dimères, les acides gras $C_{54}$-trimères (triacide), l'acide trimellitique, l'acide pyromellitique et des anhydrides correspondants.

**7.** Formulation de vernis à ongles selon la revendication 1, ledit polyol non ionique a1) étant formé en utilisant un 1,4:3,6-dianhydrohexitol en tant que molécule d'initiateur fonctionnalisée par hydroxyle dans une polymérisation par ouverture de cycle du lactide.

**8.** Formulation de vernis à ongles selon l'une quelconque des revendications 1 à 7, ledit monoalcool ou polyol a3) étant un monoalcool ou un polyol éthyléniquement insaturé réactif envers isocyanate choisi dans le groupe constitué par les (méth)acrylates de polyester, les (méth)acrylates d'époxy, les (méth)acrylates de polyéther, les (méth)acrylates de polyuréthane et les (méth)acrylates contenant un groupe hydroxyle.

**9.** Formulation de vernis à ongles selon l'une quelconque des revendications 1 à 8, ladite dispersion aqueuse durcissable de polyuréthane et ladite formulation de vernis à ongles étant une dispersion durcissable par un rayonnement ou un peroxyde.

**10.** Formulation de vernis à ongles selon l'une quelconque des revendications 1 à 9, l'au moins un composant de polyol a) possédant une teneur en poids de $^{14}C$ telle que le rapport $^{14}C/^{12}C$ est supérieur à $0,1 \times 10^{-12}$.

**11.** Formulation de vernis à ongles selon l'une quelconque des revendications 1 à 10, comprenant en outre au moins un agent colorant.

**12.** Formulation de vernis à ongles selon l'une quelconque des revendications 1 à 11, ladite formulation de vernis à ongles étant durcissable par les UV.

**13.** Formulation de vernis à ongles selon l'une quelconque des revendications 1 à 12, la formulation possédant une teneur en solides de 20 % à 30 % p/p et une viscosité de 500 mPa.s (500 cP) à 3 000 mPa.s (3 000 cP) à température ambiante.

**14.** Formulation de vernis à ongles selon l'une quelconque des revendications 1 à 13, ledit extenseur de chaîne c) portant deux groupes réactifs envers isocyanate parmi hydroxyle et amine et préférablement amine et plus préférablement l'extenseur de chaîne c) étant une diamine.

**15.** Utilisation de la formulation de vernis à ongles selon l'une quelconque des revendications 1 à 14, pour le revêtement d'ongles.

**16.** Utilisation de la dispersion aqueuse durcissable de polyuréthane telle que définie selon l'une quelconque des revendications 1 à 10, dans une formulation de vernis à ongles.

**17.** Revêtement d'ongles, résultant du durcissement d'une formulation de vernis à ongles telle que définie selon l'une quelconque des revendications 1 à 14 ou résultant d'une utilisation telle que définie selon l'une quelconque des revendications 15 et 16.

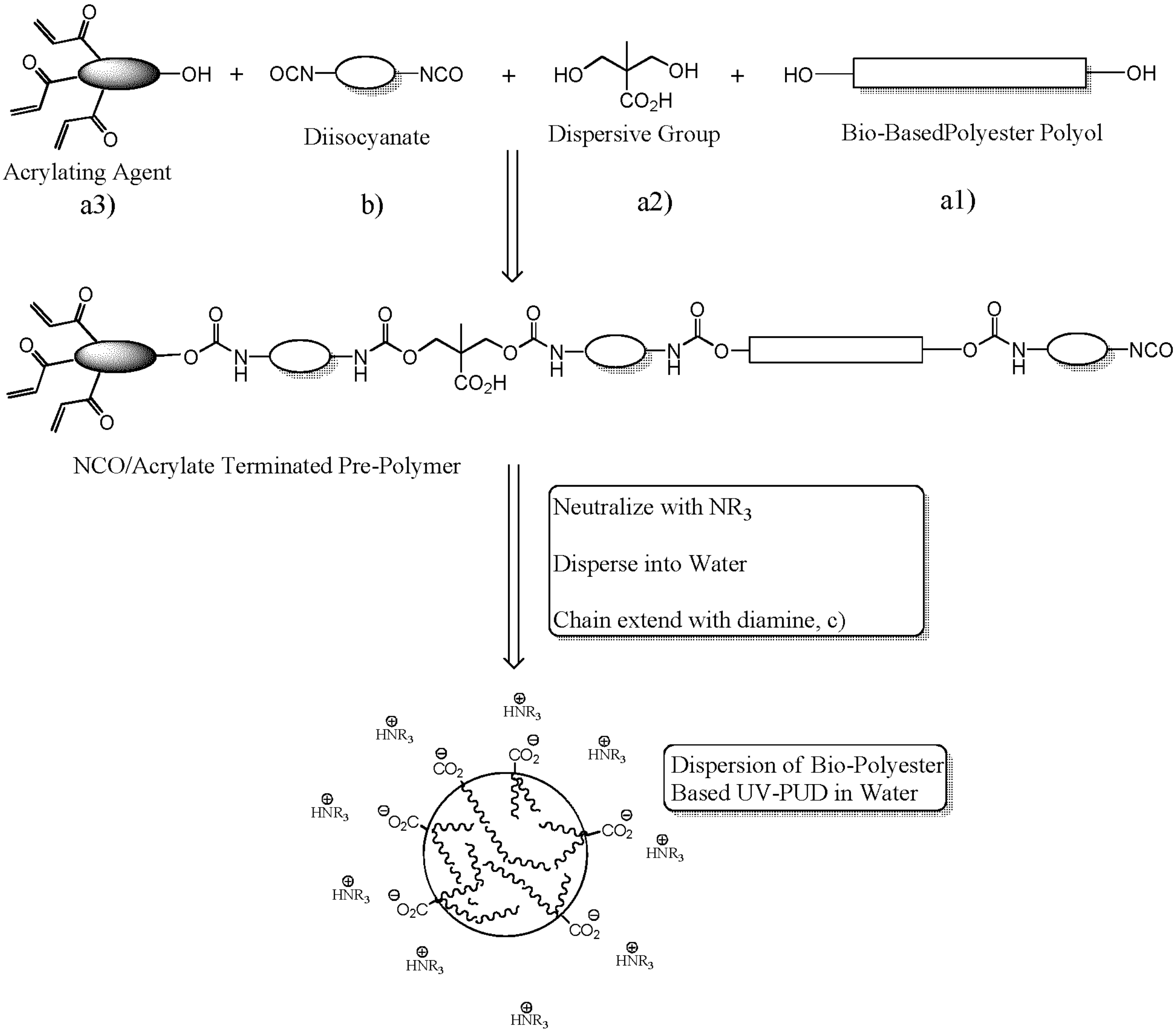
Acrylating Agent
a3)
Diisocyanate
b)
Dispersive Group
a2)
Bio-BasedPolyester Polyol
a1)
NCO/Acrylate Terminated Pre-Polymer
Neutralize with $NR_3$
Disperse into Water
Chain extend with diamine, c)
Dispersion of Bio-Polyester Based UV-PUD in Water

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011047369 A
- US 8106148 B
- WO 2011098272 A
- US 20050143549
- US 6372201 B
- US 6267950 B
- US 5716603 A
- US 20070243149 A
- US 61907434
- US 61986165


### Non-patent literature cited in the description


- **CHANG et al.** Linseed-oil-based waterborne UV/air dual-cured wood coatings. *Progress in Organic Coatings,* 2013, vol. 76, 1024-1031
- **XIA et al.** Soybean Oil-Isosorbide-Based Waterborne Polyurethane-Urea Dispersions. *ChemSusChem,* 2011, vol. 4, 386-391
- *Macromomecules,* 1976, vol. 9, 199-221